# EUROPÄISCHE PATENTSCHRIFT

(11) **EP 0 340 744 B1**
(45) Veröffentlichungstag und Bekanntmachung des Hinweises auf die Patenterteilung: **16.02.1994**
(21) Anmeldenummer: 89107979.0
(22) Anmeldetag: 03.05.1989
(51) Int. Cl.: C12P 1/00

(54) **Verfahren zur biokatalytischen Umsetzung von organischen Substanzen**
Process for the biocatalytic conversion of organic substances
Procédé de conversion biocatalytique de substances organiques

(30) Priorität: 06.05.1988 DD 315477
(43) Veröffentlichungstag der Anmeldung: 08.11.1989
(73) Patentinhaber: Degussa Aktiengesellschaft, 60311 Frankfurt (DE)
(72) Erfinder: Miethe, Peter, Dr. rer. nat., DDR-4020 Halle Saale (DD); Voss, Harald, Prof. Dr. sc. nat., DDR-4090 Halle-Neustadt (DD); Gruber, Ronald, Dipl.-Chem., DDR-4020 Halle Saale (DD)

(56) Entgegenhaltungen:
- EP-A- 0 118 928
- EP-A- 0 347 873
- EUR. J. BIOCHEM., Band 161, 1986, Seiten 149-154; N.L. KLYACHKO et al.:"Catalysis by enzymes entrapped into hydrated surfactant aggregates havinglamellar or cylindrical (hexagonal) or ball-shaped (cubic) structure in organicsolvents"

## Beschreibung

Die Erfindung betrifft ein Verfahren zur biokatalytischen Umsetzung von organischen Substanzen.

Biokatalytische Umsetzungen werden gegenwärtig überwiegend in wäßrigen oder zumindest stark wasserhaltigen Systemen ausgeführt. Diese Art der Prozeßführung ist dann ungünstig, wenn schlecht wasserlösliche bzw. wasserunlösliche, hydrophobe Substrate, z.B. Steroide, Lipide, Kohlenwasserstoffe, langkettige aliphatische oder aromatische Alkohole, Aldehyde oder Carbonsäuren, durch Biokatalyse umgesetzt werden sollen oder wenn eine enzymkatalysierte Synthese in Umkehrung einer Hydrolyesereaktion durchgeführt werden soll.

Darüber hinaus können auch Enzyme selbst stark hydrophobes Verhalten zeigen, was in wäßrigen Systemen zu einer Agglomerierung und damit zu einem Verlust der biokatalytischen Eigenschaften führt. Die bisher bekannten und verwendeten Prozeßvarianten zur biokatalytischen Umsetzung hydrophober Substrate sind:
1. Die Verwendung von Enzymen in organischen Lösungsmitteln oder wasserhaltigen Lösungsmittelgemischen.
2. Die Verwendung von zwei- und mehrphasigen Systemen.
3. Die Verwendung von einphasigen tensidhaltigen Systemen, insbesondere von reversmizellaren Phasen.

Bei Systemen vom Typ 1, besteht die Gefahr einer Enzymdenaturierung. Durch die Wahl von geeigneten Lösungsmitteln und durch die Enzymimmobilisierung kann diese lediglich verlangsamt werden. Die Standzeiten der Katalysatoren sind deshalb nur niedrig. Der Einsatz von Mikroorganismen und anderen Zellen als Biokatalysatoren ist nur bedingt möglich.

Auch bei flüssigen Systemen von Typ 2, besteht die Gefahr einer Denaturierung an der Phasengrenze. Außerdem ist die Reaktionsgeschwindigkeit aufgrund der niedrigen Substratkonzentration in der Wasserphase klein. Eine vorteilhafte Variante vom Typ 2. ist die Verwendung von hydrophilen Polymergelen zum Enzymeinschluß. Diese Polymergele, z.B. auf der Basis von Gelatine, Agar oder Alginat, können auch in organischen Lösungsmitteln eingesetzt werden. Aufgrund des hydrophilen Charakters des Polymergels ist die Konzentration des hydrophoben Substrates im Gel jedoch nur niedrig. Die Folge ist eine geringe Umsatzrate bei der Biokatalyse.

Günstigere Eigenschaften besitzen diesbezüglich Systeme von Typ 3. Reversmizellen sind globuläre Tensidaggregate, die von amphiphilen Substanzen in organischen Lösungsmitteln, beispielsweise Hexan, Heptan, Cyclohexan, Benzol, Dodecanol in Gegenwart geringer Mengen Wasser ausgebildet werden können.

Das Wasser bildet im Inneren der Mizelle einen "waterpool", in dem Enzyme eingelagert (solubilisiert) werden können. Es ist vielfach gezeigt worden, daß die Enzymaktivität dabei über längere Zeit erhalten bleiben kann. Darüber hinaus ist bekannt, daß Mikroorganismen, beispielsweise E.coli oder B.subtilis in derartigen Phasen einen Teil ihrer Enzymaktivität aufrecht erhalten können. Hydrophobe Substrate können bei den reversmizellaren Systemen im allgemeinen direckt in das organische Lösungsmittel gegeben werden.

Bei dieser Prozeßführung sind jedoch Aufarbeitungsschritte zur Rückhaltung der Tensidaggregate und zur Abtrennung der Produkte notwendig. Diese verursachen erhebliche Kosten. Eine Verbesserung der Effektivität ist bei kontinuierlicher Prozeßführung unter Verwendung immobilisierter Reversmizellen möglich. Dazu wurde ein Hohlfasermembransystem vorgeschlagen. Diese Membranen sind ein bedeutender Kostenfaktor, sie sind oft nicht lösungsmittelbeständig, neigen zur Verstopfung und stellen erhebliche Strömungswiderstände dar, so daß der Prozeß unter Druck geführt werden muß. Generell muß eingeschätzt werden, daß einphasige tensidhaltige Systeme bei einer technischen Biokatalyse äußerst problematisch sind, da insbesondere die Probleme einer einfachen Abtrennung des Produktes, der Entfernung von Tensidverunreinigungen aus dem Syntheseprodukt und der kontinuierlichen Prozeßführung bisher ungelöst sind.

Die Aufgabe der Erfindung besteht in der Umsetzung organischer Substanzen in Gegenwart einer einen Biokatalysator enthaltenden lyotropen Mesophase unter Vermeidung einer Schädigung des Biokatalysators bei kontinuierlicher oder diskontinuierlicher Prozeßführung und einfacher Abtrennung eines tensidfreien bzw. tensidarmen Produktes.

Erfindungsgemäß werden in lyotropen Mesophasen immobilisierte Biokatalysatoren zur Umsetzung von organischen Substanzen entweder in einem Zweiphasengebiet oder in einem Dreiphasengebiet eines Dreikomponentengemisches aus Wasser, organischem Lösungsmittel bzw. -gemisch und Tensid bzw. - gemisch eingesetzt. Im Zweiphasengebiet, bestehend aus lyotroper Mesophase und organischem Lösungsmittel, erfolgt die Substratzuführung und Produktabführung über die organische Lösungsmittelphase, im Dreiphasengebiet, bestehend aus lyotroper Mesophase, organischem Lösungsmittel und Wasser, erfolgt die Substratzuführung über die Lösungsmittelphase, die Produktabführung über die Wasserphase oder umgekehrt, wobei dann entsprechend Produkt bzw. Substrat wasserlöslich sein müssen.

Darüber hinaus können im Zweiphasengebiet Substrate in fester Form zugegeben und direkt von der Mesophase gelöst werden. Substrate können auch als Gas zugeführt und/oder als Gas abgeführt werden.

Ein prozeßtechnisch besonders vorteilhaftes Verfahren besteht darin, ein poröses Trägermaterial, wie Glas-und Keramiksinterkörper mit lyotroper Mesophase zu beladen. Ein solches Katalysatorsystem kann beispielsweise durch Schmelzen der Phase erzeugt werden, wobei sich nach Abkühlung die biokatalytisch aktive Mesophase in den Poren zurückbildet.

Als organische Lösungsmittel können hydrophobe Lösungsmittel, wie Hexan, Heptan, Octan, Cyclohexan, Decanol, Butylacetat, Diethylether oder Lösungsmittelgemische, wie Hexan/Aceton, Hexan/Butanol verwendet werden.

Als Tenside können anionische Tenside, wie Alkalialkylsulfate, Alkalidialkylsulfosuccinate, Erdalkalidialkylsulfosuccinate, oder kationische Amphiphile, wie Alkylammoniumsalze, Alkylpyridiniumsalze, Alkyltrimethylammoniumsalze, oder zwitterionische Amphiphile, wie Phospholipide, Sulfobetaine, Carbobetaine, oder nichtionische Amphiphile, wie Polyoxyethylenether, Polyoxyethylenester, Polyoxyethylensorbitanester, Alkylphenolpolyethylenglykolether, entsprechende Polyoxypropylenderivate oder Copolyoxyethylenpolyoxypropylenderivate oder entsprechende Tensidgemische verwendet werden.

Als Biokatalysatoren können neben isolierten Enzymen, wie Alkoholdehydrogenase, Chymotrypsin, Lipase, gegebenenfalls zusammen mit einem Coenzym, wie NAD/NADH, auch pro- und eukariontische Zellen, z.B. von E. coli oder Saccharomyces cerevisiae, oder koimmobilisierte Enzyme und Zellen eingesetzt werden. Es wurde gefunden, daß sowohl kubische, kubisch bikontinuierliche, hexagonale als auch lamellare lyotrope Mesophasen zur Solubilisierung von Biokatalysatoren geeignet sind. Dabei wird die größte biokatalytische Aktivität bei Verwendung inverser Phasen erzielt. Sowohl Zellen als auch isolierte Enzyme zeigen in einem Mehrphasensystem bestehend aus Mesophase und einer oder mehreren Lösungsmittelphasen eine hohe Aktivität. Im erfindungsgemäß verwendeten Mehrphasensystem wurde überraschend gefunden, daß die biokatalytische Aktivität im Gegensatz zu einem normalen zweiphasigen flüssigen System auch nach längeren Lagerzeiten und Reaktorstandzeiten nur wenig absinkt, daß nur sehr geringe Diffusionswiderstände in Erscheinung treten und daß die Lösungsmittelphase nur extrem wenig Tensid enthält, wodurch die Abtrennung eines nahezu tensidfreien Syntheseproduktes möglich ist.

Das erfindungsgemäße Verfahren läßt sich vorteilhaft z.B. bei der Umsetzung von organischen Substanzen, wie Steroiden, Lipiden, Kohlenwasserstoffen, langkettigen aliphatischen und aromatischen Alkoholen, Aldehyden, Carbonsäuren, Estern, Aminosäuren und Peptiden, anwenden. Der Einsatz von Biokatalysatoren für derartige Synthesen wird insbesondere bei Vorliegen von hydrophoben Substraten und Produkten wesentlich vereinfacht und verbilligt möglich. Die Biokatalyse ist dabei beeinflußt von den jeweiligen Verteilungskoeffizienten der Komponenten in den Phasen und kann über den HLB-Wert des Tensides gesteuert werden.

Bei Verwendung geeigneter, einfacher Reaktionsapparate ist es ohne weiteres möglich, das biokatalysatorhaltige flüssigkristalline System mit Hilfe mechanischer Rückhaltervorrichtungen, beispielsweise Absetzer, Separatorlamellen, Umlenkbleche oder Filter, zurückzuhalten.

Die Erfindung soll nachstehend an Ausführungsbeispielen näher erläutert werden:

### Beispiel 1:

136 ml einer 10 Gew.%-igen Lösung von Tetraethylenglykoldodecylcylether in Hexan werden mit 10 ml einer 21 mM wäßrigen NAD-Lösung versetzt und geschüttelt. In dieses zunächst trüb erscheinende dreiphasige System werden 5 ml einer wäßrigen Lösung von Hefe-Alkoholdehydrogenase und Natriumchlorid eingespritzt. Die Salzkonzentration beträgt dabei 0,1 Mol/l und die Enzymkonzentration 0,5 g/l. Nach kurzen Schütteln entsteht daraus ein zweiphasiges System, bestehend aus einer klaren viskosen lyotropen Mesophase und reinem Hexan. Dem System können beliebige Mengen an Hexan zugesetzt werden. Ein gut handhabbares System entsteht bei Zugabe von 600 ml Hexan. Bei einem Batch-Verfahren werden in dieses System ca. 15 ml Ethanol als Substrat zugespritzt. Es erfolgt die enzymatische Oxidation zum Azetaldehyd unter Bildung von NADH. Dieses ist spektralphotometrisch bei einer Wellenlänge von 365 nm in der Mesophase nachweisbar. Das System zeigt auch nach einer Lagerzeit von zwei Monaten bei Zimmertemperatur noch 80 % der Ausgangsaktivität.

Gibt man als zweites Substrat 2 ml Zimtaldehyd zu, so wird dieser unter Verbrauch des gebildeten NADH zu Zimtalkohol reduziert.

Auf diese Weise ist die Herstellung von Zimtalkohol möglich, der ohne Schwierigkeiten aus der Hexanphase, beispielsweise durch Ausschütteln mit Wasser oder anschließender Wasserdampfdestillation abgetrennt werden kann.

### Beispiel 2

Es wird in gleicher Weise verfahren, wie in Beispiel 1. An Stelle der 5 ml wäßrigen Enzymlösung wird dem System eine Suspension von Saccaromyces cerevisiae der Konzentration 10 g/l (bezogen auf Trockenmasse) zugesetzt. Der spektroskopische Nachweis des gebildeten NADH und die Produktaufarbeitung können in analoger Weise, wie in Beispiel 1 beschrieben, vorgenommen werden.

### Beispiel 3:

130 ml einer 10 Gew.%-igen Lösung von Tetraethylenglykoldodecylether in Hexan werden mit 10 ml einer 21 mM wäßrigen NAD-Lösung versetzt, anschließend werden 5 ml einer wäßrigen Lösung von Hefe-Alkoholdehydrogenase analog Beispiel 1 zugegeben. Es werden weitere 80 ml Wasser zugegeben, anschließend wird zentrifugiert. Es scheiden sich 3 Phasen, die Hexan-, die lyotrope Mesophase und eine Wasserphase ab. Die lyotrope Mesophase enthält das zugegebene Enzym. Jetzt können in analoger Weise wie in Beispiel 1 ca. 10 ml Ethanol und 2 ml Zimtaldehyd in die Hexanphase gespritzt werden. Nach etwa 20 Minuten ist in der Wasserphase spektroskopisch bei λ= 245 nm Zimtalkohol nachweisbar.

### Beispiel 4:

Es werden 136 ml einer 10 Gew.%-igen Lösung von Tetraethylenglykoldodecylether in Hexan mit 15 ml einer wäßrigen Suspension von Aspergillus glaucus versetzt. Die Konzentration der Suspension liegt dabei im Bereich von 5 - 8 g/l (bezogen auf Trockenmasse). Nach Schütteln und Zentrifugieren entsteht ein Zweiphasensystem, bestehend aus einer klaren lyotropen Mesophase und Hexan. Anschließend werden 5 ml Zimtaldehyd zugegeben. In Gegenwart von Ammoniumionen ist nach 3 Stunden Standzeit im Hexanüberstand Tyrosin nachweisbar. Der Nachweis kann dünnschichtchromatographisch auf einer Silicagelplatte, Butanol/Essigsäure/Wasser = 4/1/1 als Laufmittel erfolgen.

### Beispiel 5:

Ein weiteres zweiphasiges System, in dem lyotrope Mesophase und organisches Lösungsmittel koexistieren und in dem Biokatalysen entsprechend Beispiel 1 und 2 ausgeführt werden können, kann auf folgende Weise hergestellt werden: Man stellt eine 5 Gew.%-ige Lösung von Polyoxyethylensorbitantrioleat (Tween 85) in Heptan her. 1,4 ml dieser Lösung werden mit 800 ul Wasser oder biokatalysatorhaltiger wäßriger Lösung versetzt und geschüttelt. Es entsteht eine klare hochviskose lyotrope Mesophase, die sich im Gleichgewicht mit reinem Heptan befindet.

### Beispiel 6:

Es werden 500 ml einer 10 Gew.%-igen Lösung von Präwozell (Gemisch verschiedener Alkylphenolethoxylate, Chemische Werke Buna) in Hexan mit 40 ml einer 75 uM wäßrigen NADH-Lösung versetzt und geschüttelt. In dieses zunächst trübe dreiphasige System werden 10 ml einer 10⁻⁸ M wäßrigen Lösung von Pferdeleber-Alkoholdehydrogenase (SERVA) und 0,5 · 10⁻⁷ M Formiatdehydrogenase (SERVA) eingespritzt. Nach kurzem Schütteln entsteht ein zweiphasiges System, bestehend aus lyotroper Mesophase und Hexan. Werden als Substrat 100 µl einer prochiralen Verbindung des Typs:

CH₃ - (CH₂)ₙ - CO - CH₂ - COOCH₃ ; n = 1 - 7

in der Hexanphase gelöst, so lassen sich nach etwa 10 bis 20 Minuten die entsprechenden β-Hydroxyverbindungen gaschromatographisch nachweisen. Die Reduktion erfolgt stereospezifisch und es wird ein Enantiomerenexzeß der R-Form von 90 bis 97 % erreicht. Eine Regenerierung des verbrauchten NADH kann durch Zugabe von 200 µl Ameisensäure erreicht werden. Mit dem System sind bis zu 200 Reaktionszyklen möglich. Darüber hinaus ist auch eine Kontinuierliche Prozeßführung in einem Mixer-Settlersystem oder einem Membranreaktor möglich.

### Beispiel 7:

Zu 5 ml einer 10 %-igen Lösung von Präwozell (Gemisch verschiedener Alkylphenolethoxylate, Chemische Werke Buna) in Hexan werden 50 ml einer Suspension von Arthrobacter simplex in Phosphatpuffer (1/15 M,pH 7,0) gegeben. Nach Schütteln entsteht eine Mesophase und überstehendes Hexan. Dieses wird abgegossen und 250 ml eines Lösungsmittelgemisches aus 1 Volumenteil Toluol und 4 Volumenteilen Hexan, welches 1 mg/ml Progesteron enthält, wird zugegeben. Das System wird bei 20^{°} C mit 500 rpm gerührt. Nach 10 Stunden kann im Überstand 1-Dehydroprogesteron nachgewiesen werden. Der Nachweis kann dünnschicht - bzw. gaschromatographisch erfolgen.

### Beispiel 8:

Es wird analog zu Beispiel 7 ein zweiphasiges System hergestellt. An Stelle der wäßrigen Enzym- und Cofaktorlösung werden 50 ml einer Suspension von Pseudomonas E3 der Konzentration 20 mg/ml (bezogen auf Masse feuchte Zellen) zugesetzt. Das System wird bei 40^{°} C kontinuierlich mit einem Propen/Luft-Gemisch begast. Das entweichende Gas wird durch eine Tetrachlorkohlenstoff enthaltende Kühlfalle geleitet. Nach etwa 2 Stunden ist gaschromatographisch im Tetrachlorkohlenstoff Propylenoxid nachweisbar.

## Patentansprüche

1. Verfahren zur biokatalytischen Umsetzung von organischen Substanzen,
dadurch gekennzeichnet,
daß in lyotropen Mesophasen immobilisierte Biokatalysatoren in einem Zweiphasengebiet oder in einem Dreiphasengebiet eines Dreikomponentensystems aus Wasser, organischem Lösungsmittel bzw. Lösungsmittelgemisch sowie Tensid bzw. Tensidgemisch eingesetzt werden, wobei die Substratzuführung und Produktabführung im Zweiphasensystem, bestehend aus lyotroper Mesophase und organischem Lösungsmittel, über die Lösungsmittelphase erfolgt und im Dreiphasensystem, bestehend aus lyotroper Mesophase, organischem Lösungsmittel und wäßriger Lösung, die Zuführung von hydrophilen Substraten über die Wasserphase, die Abführung von hydrophoben Produkten über die Lösungsmittelphase oder - bei Vorliegen von hydrophoben Substraten und hydrophilen Produkten entsprechend umgekehrt - realisiert wird oder daß das Substrate in fester Form zugeführt und in der lyotropen Mesophase gelöst wird und die Produktabführung über die Lösungsmittelphase eines Zweiphasensystemes erfolgt.

2. Verfahren nach Anspruch 1,
dadurch gekennzeichnet,
daß lyotrope Mesophasen mit inverser Phasenstruktur zur Solubilisierung des Biokatalysators eingesetzt werden.

3. Verfahren nach Anspruch 1 oder 2,
dadurch gekennzeichnet,
daß als Biokatalysatoren Enzyme, pro- und eukariontische Zellen oder Enzyme in Kombination mit Zellen eingesetzt werden.

4. Verfahren nach einem der Ansprüche 1 bis 3, dadurch gekennzeichnet,
daß ein poröses Trägermaterial mit lyotroper biokatalysatorhaltiger Mesophase beladen und in dieser Form eingesetzt wird.

5. Verfahren nach einem der Ansprüche 1 bis 3,
dadurch gekennzeichet,
daß das Substrat im Zweiphasengebiet in fester Form zugegeben und direkt in der Mesophase gelöst wird.

6. Verfahren nach einem der Ansprüche 1 bis 4,
dadurch gekennzeichnet,
daß das Substrat als Gas zugeführt und/oder das Produkt als Gas abgeführt wird.

## Claims

1. Process for the biocatalytic conversion of organic substances, characterised in that biocatalysts which are immobilised in lyotropic mesophases are utilised in a two-phase region or in a three-phase region of a three-component system of water, organic solvent or solvent mixture and surfactant or surfactant mixture, wherein in the two-phase system comprising lyotropic mesophase and organic solvent the substrate is supplied and the product delivered via the solvent phase, and in the three-phase system comprising lyotropic mesophase, organic solvent and aqueous solution hydrophilic substrates are supplied via the aqueous phase, hydrophobic products are delivered via the solvent phase or - when hydrophobic substrates and hydrophilic products are present - vice versa or in that the substrate is supplied in solid form and is dissolved in the lyotropic mesophase and the product is delivered via the solvent phase of a two-phase system.

2. Process according to Claim 1, characterized in that lyotropic mesophases having inverse phase structure are utilized for the purpose of solubilising the biocatalyst.

3. Process according to Claim 1 or 2, characterized in that enzymes, prokariotic and eukariotic cells or enzymes in combination with cells are utilised as biocatalysts.

4. Process according to one of Claims 1 to 3, characterized in that a porous support material is charged with lyotropic biocatalyst-containing mesophase and is utilized in the latter form.

5. Process according to one of Claims 1 to 4, characterized in that the substrate is added in the two-phase region in solid form and is dissolved directly in the mesophase.

6. Process according to one of Claims 1 to 4, characterized in that the substrate is supplied as gas and/or the product is delivered as gas.

## Revendications

1. Procédé pour la conversion biocatalytique de substances organiques caractérisé, en ce que des biocatalyseurs immobilisés dans des mésophases lyotropes sont mis en oeuvre dans une zone à deux phases ou dans une zone à trois phases d'un système à trois composants à base d'eau, d'un solvant organique ou d'un mélange de solvants, ainsi que d'un agent tensio-actif organique ou d'un mélange d'agents tensioactifs, dans lequel l'amenée du substrat et l'évacuation du produit s'effectuent dans un système à deux phases, qui est formé d'une mésophase lyotrope et d'un solvant organique par l'intermédiaire de la phase de solvant et dans un système à trois phases qui est formé d'une mésophase lyotrope, d'un solvant organique et d'une solution aqueuse, l'amenée des substrats hydrophiles est réalisée par la phase aqueuse, l'évacuation des produits hydrophobes par la phase de solvants ou en cas de présence de substrats hydrophobes et de produits hydrophiles en sens inverse correspondant, ou bien en ce que le substrat est amené sous forme solide et est dissout dans la mésophase lyotrope et que l'évacuation du produit s'effectue par l'intermédiaire de la phase de solvant d'un système à deux phases.

2. Procédé selon la revendication 1, caractérisé en ce que l'on met en oeuvre des mésophases lyotropes avec une structure de phases inverse pour la solubilisation du biocatalyseur.

3. Procédé selon la revendication 1 ou 2, caractérisé en ce que l'on met en oeuvre comme biocatalyseur des enzymes, des cellules procaryotes et eucaryotes, ou des enzymes en combinaison avec des cellules.

4. Procédé selon l'une des revendications 1 à 3, caractérisé en ce que l'on charge un matériau support poreux avec la mésophase lyotrope contenant le biocatalyseur et qu'on la met en oeuvre sous cette forme.

5. Procédé selon l'une des revendications 1 à 4, caractérisé en ce que le substrat est ajouté sous forme solide dans la zone à deux phases et est dissout directement dans la mésophase.

6. Procédé selon l'une des revendications 1 à 4, caractérisé en ce que le substrat est amené sous forme de gaz et/ou en ce que le produit est évacué sous forme de gaz.
